# EUROPEAN PATENT APPLICATION

(11) **EP 3 656 349 A1**
(43) Date of publication of application: **27.05.2020**
(21) Application number: 18306556.4
(22) Date of filing: 22.11.2018
(51) Int. Cl.: A61D 1/00, A61B 5/00

(54) **INTRAVITAL IMAGING DEVICE**

(71) Applicant: Institut National de la Santé et de la Recherche Médicale, 75013 Paris 13 (FR); CENTRE NATIONAL DE LA RECHERCHE SCIENTIFIQUE, 75016 Paris (FR); Institut Curie, 75005 Paris (FR); Paris Sciences et Lettres - Quartier Latin, 75006 Paris (FR)
(72) Inventor: JACQUEMIN, Guillaume, 37000 Tours (FR); FRE, Silvia, 75004 Paris (FR); LLOYD-LEWIS, Bethan, 75014 Paris (FR); JACQUEMIN, Philippe, 56230 Questembert (FR)
(74) Representative: IPAZ

(57) **Abstract**

The invention relates to an Intravital Imaging Device (IID) comprising:
- a window forming at least a part of a face of the IID,
- a frame extending outward from a contour of the window, the frame comprising:
• an inner bulge located on a side of the IID comprising the window,
• an outer bulge located on a side of the IID opposite to the side of the IID comprising the window,
• a groove located between the outer and inner bulge.

The IID is arranged to reversibly decrease and/or increase a surface defined 10 by a perimeter in which the IID is inscribed.

## Description

### Field of the invention

The present invention relates to an Intravital Imaging Device (IID). IIDs are used to carry out Intravital Microscopy (IM) in living animals. IM is a technique by which internal tissues are imaged over time, for instance to study physiological, pathophysiological and therapeutic processes through long-term experiments. This technique is increasingly utilized to avoid resorting to surgical dissection and its drawbacks such as tissue dehydration, impaired thermoregulatory control and/or animal survival, possible effects of prolonged anesthesia exposure and a limited field of view.

### Background to the invention

Prior art discloses few different types of IID. Conventional types of IID consist of a glass window coverslip inserted in a frame that protects and gives optical access to the tissue to be imaged. One example of such devices is described by Alieva, M. et al., (2014); Imaging windows for long-term intravital imaging: General overview and technical insights; Intravital 3, e29917*.* Glasses are usually treated with Polyethylene Glycol to reduce inflammation and cell adhesion. Frames are usually made of titanium. IIDs have to exhibit common characteristics such as a suitable biocompatibility and a window having a value of refractive index close to that of water.

IIDs according to the state of the art require on sutures, generally purse-string, to be implanted. Usually an incision above the organ/tissue to be imaged is provided, then the window is inserted through this latter and sutures to tighten the skin around the frame are carried out. The need to resort to sutures combined to the high weight of current IIDs lead to animal pain and stress, skin degradation, inflammation/fibrosis (which precludes imaging) and window detachment, especially in contexts where animals undergo significant body or tumour growth during the study timeframe. These issues frequently result in the premature termination of experiments.

### Summary of the invention

An object of the invention is to provide:
- an IID that exhibits a suitable biocompatibility and comprises a window having a low value of refractive index, and/or
- an IID having a window with high value of light transmittance, and/or
- an IID that exhibits a low weight, and/or
- an IID that can be implanted in a suture-free manner, and/or
- an IID that can be seamlessly implanted.

To this end, according to a first aspect of the invention, there is provided an Intravital Imaging Device (IID) comprising:
- a window forming at least a part of a face of the IID,
- a frame extending outward from a contour of the window, the frame comprising:
   µ an inner bulge located on the side of the frame comprising the window,
   µ an outer bulge located on a side of the IID opposite to the side of the IID comprising the window,
   • a groove located between the outer and inner bulge.

The IID according to the invention is characterized in that, it is arranged to reversibly decrease and/or increase a surface defined by a perimeter in which the IID is inscribed.

The term "a surface defined by a perimeter in which the IID is described", may be understood as an area of a surface inscribed into an outer contour of the IID that is projected onto a plane. In particular, but not exclusively, the plane may be the plane in which the contour of the window is inscribed. The term "a surface defined by a perimeter in which the IID is described", may be also understood as a framework in which the IID is inscribed.

The IID may also be defined as being arranged to reversibly decrease and/or increase a size of the frame and/or a size of the inner bulge and/or a size the outer bulge and/or a size of the window and/or a size of the groove.

The IID may also be defined as being arranged to reversibly decrease and/or increase a length of at least one segment connecting two points of the outer contour of the IID and/or the outer contour of the windows and/or outer contour of the of the frame and/or the groove and/or the inner bulge and/or the outer bulge.

The face of the IID comprising the window may be located on a side of a frame of the IID intended to face an area to be imaged.

The outer bulge and/or the inner bulge may be extending outward:
- on the side of the face of the IID comprising the window, or
- on the side of the IID opposite to the side of the face of the IID comprising the window.

The frame may comprise one or more walls extending from the window towards an opening of the frame, said opening being located on a side of the frame opposite to the side of the frame comprising the window.

The groove may be intended to receive a tissue of an host.

The groove may be defined as being formed between the outer and the inner bulges.

The side of the frame of the IID being intended to face the area to be imaged may be arranged to be brought in contact with said area to be imaged.

According to the invention, the area to be imaged may be also defined as an object to be imaged. The object may be all type of organic object.

The area may comprise a tissue or an organ or any part of an animal.

The term "animal" according to the invention may refers to a mammal such a rodent.

The one or more walls of the frame may form a duct. The duct may comprise apertures or openings.

The one or more walls may not be continuous; these discontinuities may form apertures or openings.

The opening of the frame may constitute a face of the frame opposite to the side of the frame comprising the window.

The opening may also be defined as an opening of the IID.

According to the invention, a bulge may also be defined as a protrusion or an annular bead.

The inner bulge may form a part of the face located on the side of the frame comprising the window.

The outer bulge may abut the opening.

The groove may also be defined as being formed by the outer and the inner bulges.

The IID may be arranged to reversibly decrease and/or increase, according to at least a direction parallel to the face of the IID comprising the window, the size of the frame and/or the size of the inner bulge and/or the size the outer bulge and/or the size of the window and/or the size of the groove.

The IID may be arranged to reversibly decrease and/or increase a distance between two of its parts and/or a distance between two points of the frame and/or a distance between two points of the inner bulge and/or a distance between two points the outer bulge and/or a distance between two points of the window and/or a distance between two points of the groove.

The IID may be arranged to reversibly decrease and/or increase, according to at least a direction parallel to the face of the IID comprising the window, a distance between two of its parts and/or a distance between two points of the frame and/or a distance between two points of the inner bulge and/or a distance between two points the outer bulge and/or a distance between two points of the window and/or a distance between two points of the groove.

At least a part of the inner bulge and/or at least a part of the IID and/or at least a part of the frame and/or at least a part of the outer bulge and/or at least a part of the window and/or at least a part of the groove may be reversibly retractable or extend.

At least a part of the inner bulge and/or at least a part of the IID and/or at least a part of the frame and/or at least a part of the outer bulge and/or at least a part of the window and/or at least a part of the groove may be reversibly retractable or extend at least towards the center of the window and/or at least towards a direction parallel to the face of the IID comprising the window.

The entire inner bulge and/or the entire IID and/or the entire frame and/or the entire outer bulge and/or the entire window and/or the entire groove may be reversibly retractable or extend.

The entire inner bulge and/or the entire IID and/or the entire frame and/or the entire outer bulge and/or the entire window and/or the entire groove may be reversibly retractable or extend at least towards the center of the window and/or to at least towards a direction parallel to the face of the IID comprising the window.

At least a part of the inner bulge and/or at least a part of the IID and/or at least a part of the frame and/or at least a part of the outer bulge and/or at least a part of the window and/or at least a part of the groove may be mobile at least relatively to the center of the window. The term "mobile" may be understood as can be moved.

The entire inner bulge and/or the entire IID and/or the entire frame and/or the entire outer bulge and/or the entire window and/or the entire groove may be mobile at least relatively to the center of the window.

The face of the IID comprising the window may exhibit a flexible size. The term "flexible" can be understood as variable, adjustable, modular or adaptable.

The size of the frame and/or the size of the window and/or the size of the inner bulge and/or the size of the IID and/or the size of the outer bulge and/or the size of the groove may exhibit a flexible size.

The face of the IID comprising the window may exhibit a flexible size according to at least a direction parallel to said face.

The size of the frame and/or the size of the window and/or the size of the inner bulge and/or the size of the IID and/or the size of the outer bulge and/or the size of the groove may exhibit a flexible size according to at least a direction parallel to the face of the IID comprising the window.

According to the invention:
- a non-zero angle may be formed between a plane in which the contour of the window is inscribed and an axis along which the outer bulge is extending, and/or
- a non-zero angle may be formed between the plane in which the contour of the window is inscribed and an axis along which the inner bulge is extending.

The axis along which the inner bulge is extending may be defined as the axis along which it is extending outward from the center of the IID. The axis along which the outer bulge is extending may be defined as the axis along which it is extending outward from the center of the IID.

According to the invention:
- a non-zero angle may be formed between a surface along which the face of the IID comprising the windows is extending and a surface along which the outer bulge is extending, and/or
- a non-zero angle may be formed between a surface along which the face of the IID comprising the windows is extending and a surface along which the inner bulge is extending.

A width of the groove may decrease along a direction extending outward from a bottom of the groove.

The width of the groove may also be defined as the distance between the outer and the inner bulge.

Preferably, the width may be maximum at the bottom of the groove and may decrease along the direction extending outward from the bottom of the groove.

Preferably, the width may be maximum on the side of the groove located towards the center of the IID and may decrease along the direction extending outward from the center of the IID.

The inner and the outer bulge may be arranged to act as a clamp.

The inner and the outer bulge may be arranged to exerting a clamping force to prevent the tissue to be pulled out from the groove.

A surface defined by a perimeter in which the inner bulge is inscribed may be larger than a surface defined by a perimeter in which the outer bulge is inscribed or vice versa.

The inner bulge may be inscribed in a framework larger than a framework in which the outer bulge is inscribed.

The bulges may also be described as being within or being included or being enclosed in a framework. In this respect, a framework may also be understood as a frame. The framework may also be understood as a closed line within which the bulge is inscribed.

The inner bulge may occupy a space larger than a space occupy by the outer bulge. The inner bulge may occupy a space larger, according to at least a given direction being parallel to the face of the IID comprising the window, than a space occupy by the outer bulge.

A distance between the one or more walls and an outer edge of the inner bulge may be larger than a distance between the one or more walls and an outer edge of the outer bulge.

According to the invention, an outer edge may be understood as an end edge or an outer rim or an outer end.

A distance between the one or more walls and the outer edge of the inner bulge may be larger, according to at least a given direction being parallel to the face of the IID comprising the window, than a distance between the one or more walls and the outer edge of the outer bulge.

A distance between a central axis and the outer edge of the inner bulge may be larger than a distance between the central axis and the outer edge of the outer bulge; said central axis being normal to the face of the IID comprising the window and passing through the center of the window.

A distance between the central axis and the outer edge of the inner bulge may be larger, according to at least a given direction being parallel to the face of the IID comprising the window, than a distance between the central axis and the outer edge of the outer bulge.

A perimeter of the inner bulge may be larger than a perimeter of the outer bulge. The perimeter may be understood as a contour.

A radius of the inner bulge may be larger than a radius of the outer bulge, a radius of a bulge being the distance extending from a wall of the IID towards the outer edge of the bulge. The radius of a bulge may also be described as the distance extending from a wall of the IID towards the outer edge of the bulge according to a direction normal to the wall of the duct and/or according to a direction normal to the central axis.

At least one protrusion may be extending from the outer edge of the inner bulge.

At least one protrusion may be extending from the outer edge of the inner bulge towards at least a direction extending from the center of the window towards the outer edge of the inner bulge.

The at least one protrusion may be in the form of a strip or a tab.

The IID may exhibit at least two states:
- a retracted state, and
- an extended state wherein an apparent size of the window is higher than an apparent size of the window when the IID is in its retracted state.

The retracted state may also be defined as a compacted state or a folded state. The extended state may also be defined as an unfolded state, an elongated state, an unpacted state or an stretched state. An apparent size of the window may also be defined as a visible part of the window by which the object to be imaged is visible.

The frame and/or the window may be made of flexible material.

Flexible may be understood as bendable, pliable, pliant.

According to the invention, when no external force is applied to the IID, or to a part of the IID, the IID may tend to:
- return in its extended state, or
- return in its retracted state, or
- stay in the state wherein it has been brought, the state may be extended, retracted or any other state comprises between the retracted and extended states.

When at least a part of the IID is flexible, the IID may tend to return in an idle state. In the idle state, the apparent size of the window may be comprises between:
- the minimum apparent size of the window when the IID is in its retracted state, and
- an apparent maximum size of the window.

An idle state may be understood as a steady state or a stable state.

When at least a part of the IID is flexible, a size of the window may be larger than the size of the window in its extended state. When no part of the IID is flexible, the IID may exhibit at least two states, a retracted state wherein an apparent size of the window is minimum and an extended state wherein an apparent size of the window is maximum.

In a first embodiment of the first aspect of the invention, the frame may comprise rigid portions, two adjacent portions may be joined together about one of their ends. Join may be understood as connected or bound. According to the first embodiment, the window is flexible.

Rigid may be understood as non-flexible in term of elasticity and deformation capabilities. Unlike a flexible element, a rigid element exhibits a high Young's modulus.

The rigid portions may be jointed together through hinges, two adjacent portions being hinged together about one of their ends. Portions may be understood as parts, sections, segments or pieces.

The window may be stuck or glued to, or molded or overmolded on, the frame.

According to an alternative, each portion may comprise a housing, or a slot, in which the window, or a part of the window, is inserted. The window may be stuck or glued, or molded or overmolded, within the housing of the frame.

In a second embodiment of the first aspect of the invention, the frame may comprise portions separated from one another.

According to a first alternative of the second embodiment of the first aspect of the invention, the window may be flexible.

According to the first alternative, the frame may be rigid or flexible.

According to the first alternative, the window may be stuck or glued to, or molded or overmolded on, the frame.

According to the first alternative, each portion may comprise a housing, or a slot, in which the window, or a part of the window, is inserted.

According to the first alternative, when the portions comprise a housing, or a slot, the window may be stuck or glued, or molded or overmolded, within the housings of the portions.

According to a second alternative of the second embodiment of the first aspect of the invention, the window may be rigid.

According to the second alternative, the frame may be rigid or flexible.

According to the second alternative, each portion comprises a housing, or a slot, in which the window, or a part of the window, is inserted.

According to the second alternative, the housings may be arranged for the portions to be moved relative to the window through sliding of the window inside the housings.

According to the second alternative, when the frame is rigid:
- the window may be fully inserted in the housings when the IID is in its retracted state,
- the window may be partially inserted in the housings when the IID is in its extended state.

In a third embodiment, the frame may comprise two end edges or two end sides and the frame may be flexible so that the two end edges may be mobile relatively from one another.

Each end edge or each end side is elastically bound to another part of the frame. The two end edges or the two end sides may be elastically bound to one another.

The elastic bound may tend to bring the IID:
- in the retracted state wherein an apparent size of the window is minimum, and/or
- in the extended state wherein an apparent size of the window is is larger than the size of the window in its retracted state.

The frame may comprise a housing or a slot in which the window, or a part of the window, is inserted.

In a first alternative of the third embodiment, the window may be flexible. The window may be stuck or glued to, or molded or overmolded on, the frame. The window may be stuck or glued, or molded or overmolded within the housing of the frame.

In a second alternative of the third embodiment, the window may be rigid.

According to the second alternative of the third embodiment, the IID may be:
- in its retracted state, an outer edge surface s1 of the window is covered by the frame,
- in its extended state, an outer edge surface s2 of the window, smaller than si, is covered by the frame.

When the IID is in its retracted state, the window is fully inserted in the housing. When the IID is in its extended state, the window is partially inserted in the housing.

The frame and the window may be flexible. Flexible may be understood as stretchy.

The frame and the window may be reversibly deformable.

The frame and the window may be elastic.

The frame and the window may be made of flexible material. The frame and the window may consist of several flexible materials. The frame and the window may consist of a unique flexible material.

The window may be made of any transparent material that exhibits elastic properties.

The frame and the window may comprise a mineral polymer and/or an organic polymer and/or wax and/or glass.

The frame and the window may comprise one or more elastomer. The frame and the window may be made of elastomer. The frame and the window may comprise PolyDiMethylSiloxane (PDMS). The frame and the window may be made of PDMS. The frame and the window may comprise and/or may be made of polymeric organosilicons.

The frame and the window may form a single piece. The frame and the window may be integrally formed.

The frame and the window may be integrally formed in the same material. The frame and the window may be integrally formed in a single material.

The frame and the window may be integrally formed using two or more different materials by molding and/or overmolding and/or gluing.

The inner bulge may comprise:
- a coating and/or a functionalization and/or a surface treatment and/or a filler material to promote the healing of the host and/or to promote the adhesion of a tissue of the host, and/or
- a surface pattern and/or an increase of surface roughness to promote the healing of the host and/or to promote the adhesion of the tissue of the host. A surface pattern may be understood as surface comprising structures, such as micro and/or nanostructures.

The filler material may comprise degradable beads arranged to generate 3D structures and/or hydrophobic drugs inclusion to deliver drugs, for instance anti-inflammatory treatment.

The groove and/or the outer bulge and/or the window and/or the frame or the entire IID may comprise:
- a coating and/or a functionalization and/or a surface treatment and/or a filler material to promote the healing of the host and/or to promote the adhesion of the tissue of the host, and/or
- a surface pattern and/or an increase of surface roughness to promote the healing of the host and/or to promote the adhesion of the tissue of the host.

The outer bulge may comprise means for protecting the IID against animal degradation.

The means for protecting IID against animal degradation may comprise:
- an abrasion resistant shield, and/or
- a degradable distracting structure to attract animal attention. The degradable distracting structure seeks to prevent the outer bulge degradation. The abrasion resistant shield may resist to friction and/or bite and/or pull. The degradable distracting structure is deliberately visible and/or deliberately reachable by the host for the degradable structure to catch or focus the host attention and to protect the bulges and/or the frame from animal degradation.

The IID may comprise a housing located on the face of the IID comprising the window, the housing being arranged for:
- receiving an object of the host to be imaged, and/or
- immobilizing the object of the host to be imaged, and/or
- conserving the object to be imaged.

The housing may be adjacent to the window.

The housing may receive the object to be imaged. The housing may be a structure, such as a groove and/or an opening and/or a recess provided in the window and/or micro-structures. The housing may be arranged for the organ to be imaged to be inserted in it.

The housing may form a single piece with the IID.

The housing may be an additional part glued and/or nested to the IID core.

The frame may form a protrusion extending beyond the window towards the area to be imaged. The protrusion may comprise the housing. The protrusion may comprise an opening extending from the housing beyond an end of the protrusion located on the side of the protrusion opposite to the window.

According to a second aspect of the invention, there is also provided a method for implanting an Intravital Imaging Device (IID) according to the invention, whereby the method comprises the steps consisting of:
- retracting the IID by reversibly decreasing a surface defined by a perimeter in which the IID is inscribed,
- loading the retracted IID into a pipe through an opening of the pipe,
- arranging the opening so that it faces the area to be imaged of an host,
- applying a pressure in the pipe, said pressure being directed towards the opening for releasing the IID from the pipe so as:
   - a groove of the IID receives a tissue of the host, and
   - a window faces the area to be imaged.

The IID may be folded.

The IID may be retracted so as to decrease an area of a surface inscribed into an outer contour of the IID that is projected onto a plane in which a contour of the window is inscribed before the retracting step. The IID may be retracted so as to decrease a length of at least one segment connecting two points of the outer contour of the IID and/or the outer contour of the windows and/or the frame and/or the groove the inner bulge and/or the outer bulge.

The IID may be inserted into the pipe through the opening of the pipe. As non-limiting examples, the pipe may be a tube or a syringe-type tube or a duct. The IID acts as a clog after being loaded. Encircle may be understood as clasp or surround. The tissue may firmly encircle the groove.

The IID to implant through the method according to the invention, may comprises a frame and one or more walls, the groove may be located between an outer and an inner bulge, the window may form at least a part of a face of the IID and a distance between the one or more walls and an outer edge of the inner bulge is larger than a distance between the one or more walls and an outer edge of the outer bulge according to at least a given direction being parallel to the face of the IID comprising the window.

The IID to implant through the method according to the invention may be the IID as described according to the first aspect of the invention. Any feature, alternative and embodiment of the first aspect of the invention may be associated with the second aspect of the invention if they are not mutually exclusive of each other.

The method according to the second aspect of the invention, wherein:
- after the step of retracting at least a part of the IID according to at least a direction parallel to the face of the IID that comprises the window, the IID may be in a retracted state wherein an apparent size of the window is minimum,
- after the step of releasing the IID from the pipe, the IID may be in an extended state wherein an apparent size of the window is larger than the size of the window in its retracted state.

The method according to the second aspect of the invention may comprise the step consisting in arranging the opening into an incision site of a tissue of the host. The incision site may be defined as a pre-incised site.

As non-limiting examples, the tissue may be skin or a biological tissue or any organic tissue.

The method according to the second aspect of the invention may comprise the step consisting in inserting a rounded rim of the pipe into the pre-incised site of the tissue and arranging the rounded rim between the area to be imaged and the tissue; the rounded rim being abutted on outer edges of said opening.

The rounded rim of the pipe may form an annular protrusion. The term "abuted" may be understood as "contiguous".

The pipe may comprise two rims arranged for the tissue to encircle the tube in an area of the tube located between the two rims.

The method according to the second aspect of the invention wherein, subsequently to the retracting step:
- a clamping force may be applied on the tissue of the host located inside the groove, and/or
- a lifting force may be applied by the IID on the tissue of the host located outside the groove so that a part of the tissue of the host is positioned above and/or covers an outer peripheral portion of the IID.

According to a third aspect of the invention, there is also provided a method for preclinical testing or preclinical study or in vivo drug screening, said method comprising the steps consisting of:
- retracting the IID by reversibly decreasing a surface defined by a perimeter in which the IID is inscribed,
- loading the retracted IID into a pipe through an opening of the pipe,
- arranging the opening so that it faces the area to be imaged of an host,
- applying a pressure in the pipe, said pressure being directed towards the opening for releasing the IID from the pipe so as:
   - a groove of the IID receives a tissue of the host, and
   - a window faces the area to be imaged.

The method according to the third aspect of the invention may comprise any combination of any feature of the method according to the second aspect of the invention.

The IID 1 may comprise any combination of any feature, improvement, alternative or embodiment of the IID 1 according to the invention.

The method according to the third aspect of the invention may further comprise the steps of:
- intravital imaging of the area to be imaged, and/or
- administering the candidate compound to the animal.

### Brief description of the drawings

Further objects, features and advantages will appear from the following detailed description of several embodiments of the invention with references to the drawings, in which:
- FIGURE 1a is a schematic representation of an oblique view of a first embodiment of the IID,
- FIGURE 1b is a schematic representation of a side view of the first embodiment of the IID,
- FIGURE 1c are schematic representation of side and oblique views of a the IID is its retracted state,
- FIGURES 2a and 2b are schematic representations of a side view of an improvement of the IID according to the invention,
- FIGURE 3 is a schematic representation of a variant of the inner bulge according to the invention,
- FIGURES 4a and 4b are schematic representations of side views of means for protecting the IID against animal degradation,
- FIGURE 5 are schematic representations of oblique and side views of geometric variants of IIDs according to the invention,
- FIGURES 6a and 6b are schematic representations of respectively side and top views of an IID comprising a housing to immobilize and/or conserve an object to be imaged,
- FIGURES 7a and 7b are schematic representations of portions of the IID according to a first embodiment,
- FIGURE 8 are schematic representations of the first embodiment of the IID,
- FIGURE 9a is a schematic representation of a portion of the IID according to a second embodiment,
- FIGURE 9b is a schematic representation of a top view of the second embodiment of the IID,
- FIGURE 10 are schematic representations of top views of a third embodiment of the IID,
- FIGURE 11 is a schematic representation of a side view of an embodiment of the method according to the invention.

### Detail description of embodiments of the invention

The embodiments hereinafter described are not restrictive, other embodiments comprising a selection of features described hereinafter may be considered. A selection may comprise features isolated from a set of features (even if this selection is isolated among a sentence comprising other features thereof), if the selection is sufficient to confer a technical advantage or to distinguish the invention form the state of the art. This selection comprises at least a feature, preferably described by its technical function without structural features, or with a part of structural details if this part is sufficient to confer a technical advantage or to distinguish the invention form the state of the art on its own.

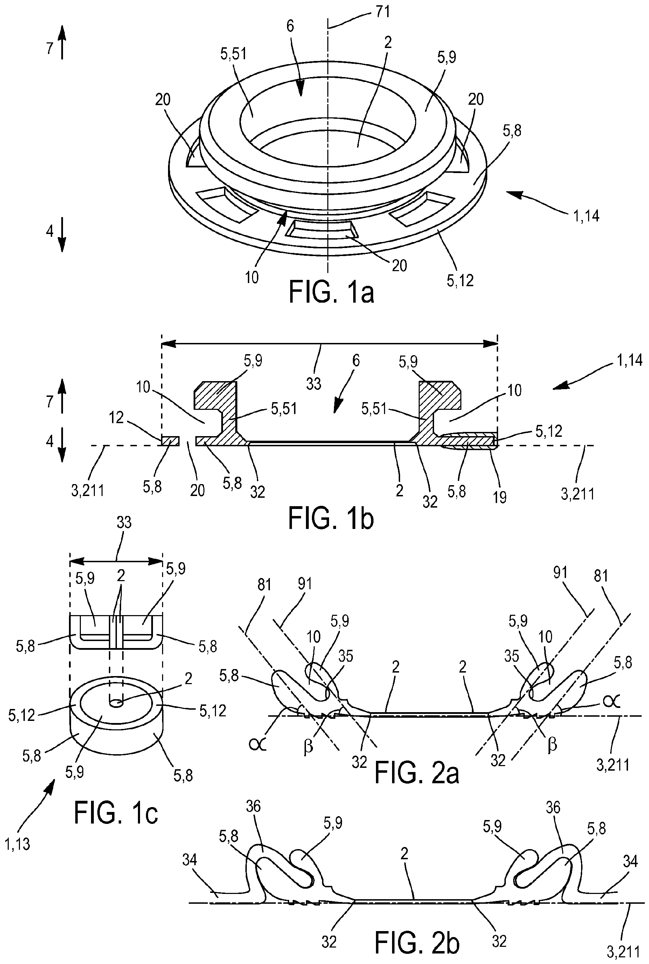

With reference to FIGURES 1a, 1b, 1c, 2a, 2b, 3, 4a, 4b, 5, 6a and 6b, an Intravital Imaging Device (IID) 1 according to the invention is illustrated.

With reference to FIGURE 1b, the IID 1 comprises a window 2 forming at least a part of a face 3 of the IID 1. The face 3 of the IID 1 comprising the window 2 is located on a side 4 of a frame 5 of the IID 1 that is intended to face the area to be imaged. The frame 5 extends outward from a contour 32 of the window 2The frame 5 comprises an inner bulge 8 located on the side 4 of the frame 5 that comprises the window 2. The frame 5 comprises an outer bulge 9 located on a side 7 of the IID 1 opposite to the side 4 of the IID 1 comprising the window 2The frame 5 comprises a groove 10 located between the outer bulge 9 and the inner bulge 8.

According to the invention, the IID 1 is arranged to reversibly decrease and/or increase a surface 33 defined by a perimeter in which the IID 1 is inscribed. The surface 33 can also be defined as the area 33 of the surface inscribed into an outer contour 12 of the IID 1 that is projected onto a plane parallel to the plane in which the contour 32 of the window 2 is inscribed. According to the invention, the outer contour of the IID 1 is the outer edge 12 of the inner bulge 8. Another way to described this feature is that the IID 1 is arranged to reversibly decrease and/or increase its size and/or a size of the frame 5 and/or a size of the inner bulge 8 and/or a size the outer bulge 9 and/or a size of the window 2 and/or a size of the groove 10. Each of these different combinations are detailed hereinafter through embodiments illustrated Figures 1a, 1b, 1c, 2a, 2b, 6a, 6b, 7, 8a, 8b and 9.

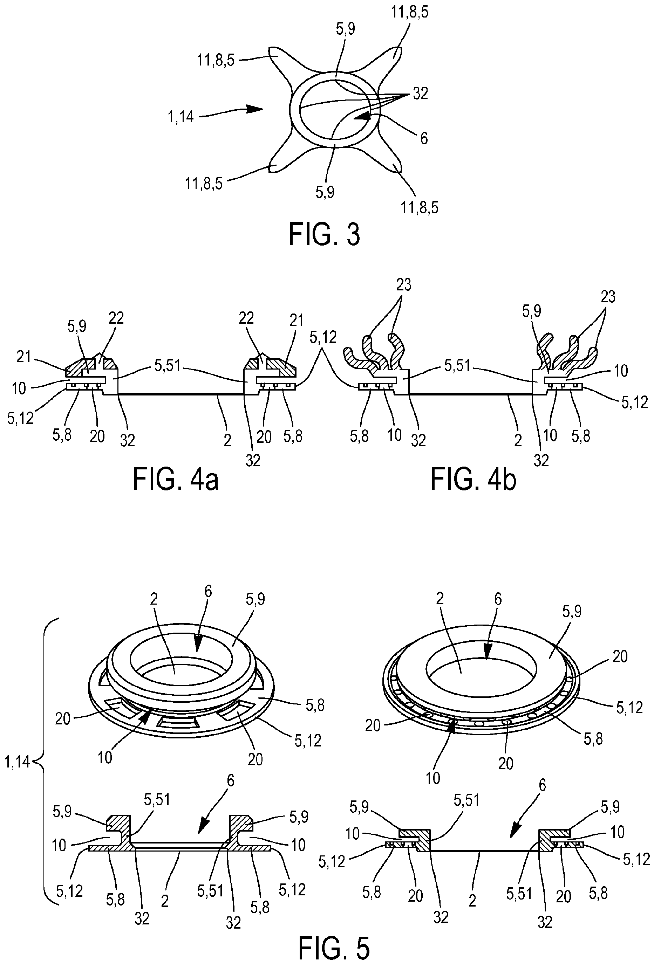

According to the invention, when the IID 1 is implanted, the tissue of the animal is inserted into the groove 10. Thus, the tissue surrounds and encloses the groove 10. According to the invention, the inner bulge 8 is larger than the outer bulge 9. This feature enables the tissue of the animal to apply a constant pressure onto the inner bulge 8 sufficient for the IID 1 to be held in contact against the area to be imaged. When the groove 10 is not sufficiently deep, and/or the inner bulge 8 not sufficiently large, the applied pressure is not sufficient for the IID 1 to be held in contact against the area to be imaged. Moreover, in this case, the surface of the tissue in contact with the inner bulge 8 is small. Therefore, the tissue is damaged by pulling forces exerted by the IID 1, and/or the object to be imaged is damaged by frictions of the IID 1 on it. This feature also avoids resorting to sutures to maintain the IID 1. Indeed, the bearing surface of the tissue applying a pressure onto the inner bulge 8 is sufficient to efficiently immobilize the IID 1 against the area to be imaged. When the IID 1 exhibits other geometrical shapes, and in order to achieve these technical effects, the inner bulge 8 can be described as being inscribed in a framework larger than a framework described by the outer bulge 9. By way of a non-limiting example, a further embodiment of the feature by which the inner bulge 8 is larger than the outer bulge 9 is illustrated FIGURE 3. One can see that the inner bulge 8 of the IID 1 comprises strip-shaped protrusions 11 extending from the outer edge 12 of the inner bulge 8 towards a direction normal to the imaging direction 71. These protrusions 11 are intended to be inserted under the tissue of the animal located above the area to be imaged.

According to the invention, the apparent size of the window 2 varies between the minimum size for which the IID 1 is in its retracted state 13 and a size larger than the apparent minimum size wherein the IID 1 is in its extended state 14. In some embodiment, the minimum apparent size of the window 2 for which the IID 1 is in its retracted state 13 is zero.

In reference to FIGURES 2, it is presented an improvement of the IID 1 according to the invention compatible with all the embodiments. One can see that a non-zero angle β is formed between a plane 211 in which the contour 32 of the window 2 is inscribed and an axis 91 along which the outer bulge 9 is extending. A non-zero angle ϕ is also formed between the plane 211 in which the contour 32 of the window 2 is inscribed and an axis 81 along which the inner bulge 8 is extending. The angle β is equal to the angle ϕ. According to the improvement, the bulges 8,9 are extending outward on the side of the IID 1 opposite to the side of the face 3 of the IID 1 that comprises the window 2. This arrangement of the IID 1 allows, after implantation of the IID 1, a portion 36 the tissue 34, in most cases the skin 34, of the organ of the animal to be imaged to lifted above the top of the bulges 8,9. After implantation, the skin 34 covers, or overlay, the bulges 8,9 so that the IID 1 does not exhibit any part accessible by the animal. This arrangement prevents the animal from attempting to remove the IID 1 and so promotes the healing of the animal and allows long term experiment.

According to an improvement of the IID 1, the width of the groove 10 decrease along a direction extending outward from a bottom 35 of the groove 10. The width of the groove 10 corresponds to the distance between the faces of the bulges 8,9 facing each other. The groove 10 receives the tissue 34 during the implantation. The decrease of the width of the groove 10 allows the tissue 34 to be pinched by the bulges 8,9 after implantation. This prevents the tissue 34 to be pulled out from the groove 10 after implantation.

According to an improvement of the IID 1, the inner 9 and the outer bulge 10 are arranged to act as a clamp. For instance, in order for the bulges 8,9 to exert a clamping force, the Young modulus of the material, in which the frame 5 and/or the IID 1 are made of, is comprised between 0,1 MPa and 10 GPa.

When the IID 1 is flexible, the apparent size of the window 2 is comprised between the minimum apparent size of the window 2 for which the IID 1 is in its retracted state 13 and the maximum apparent size of the window 2.

When the IID 1 is rigid, the apparent size of the window 2 is comprised between the minimum apparent size of the window 2 for which the IID 1 is in its retracted state 13 and the maximum apparent size of the window 2 for which the IID 1 is in its extended state 14.

After being implanted, the tissue can cause the IID 1 to:
- tend to be in its retracted state 13, or
- decrease the size of the window 2 towards a size smaller than the size for which the IID 1 is in its extended state 14.

When the tissue causes the size of the window 2 to change, an external force may be manually applied during the imaging to bring the IID 1 in its extended state 14 to provide a larger view of the object to be imaged.

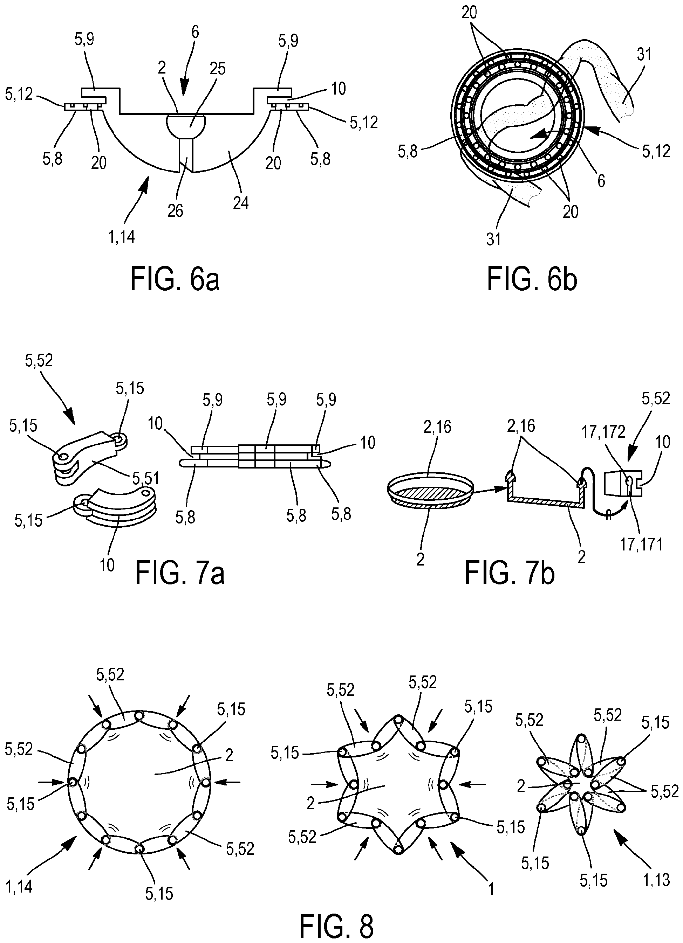

In some embodiments, the frame 5 comprises a wall 51 extending from the window 2 towards an opening 6 of the frame 5. The wall 51 is circular. The opening 6 is located on a side 7 of the frame 5 opposite to the side 4 of the frame 5 that comprises the window 2. With reference to FIGURES 1, 4 and 5, the size of the opening 6, according to a direction normal to the direction whereby the opening is extending, said imaging direction 71, is equal to the size of the window 2 according to a direction normal to the imaging direction 71. With reference to FIGURE 6, the size of the opening 6, according to a direction normal to the imaging direction 71, is smaller than the size of the window 2 according to a direction normal to the imaging direction 71. The frame 5 comprises an outer bulge 9 located between the inner bulge 8 and the opening 6 of the IID 1.

In a first embodiment depicted in FIGURES 7 and 8, the frame 5 comprises rigid portions 52 and the window 2 is flexible. Two adjacent portions 52 are joined together at one of their ends through swivel 15 provided at ends of the portions 52. Two rigid portions 52 swivel relative to one another along a direction normal to the imaging direction 71.

The window 2 comprises an annular bead 16 which is stuck or glued, or molded or overmolded, within the housings 17. One housing is provided in each rigid portion 52 of the frame 5. The housing 17 comprises a slot 171, provided in the rigid portion 52. A recess 172 is arranged at the end of the slot 171. The annular bead 16 is held in the recess 172.

When the IID 1 is brought in its retracted state 13, two adjacent rigid portions 52 swivel and get closer to one another. The window 2 is then folded.

The IID 1 exhibits no idle state. When no external force is applied to the IID 1, or to a part of the IID 1, the IID 1 tends to stay in the state wherein it has been brought. According to the first embodiment, the apparent size of the window 2 when the IID 1 is in its retracted state 13 is the minimum apparent size of the window 2 and the apparent size of the window 2 when the IID 1 is in its extended state 14 is the maximum apparent size of the window 2.

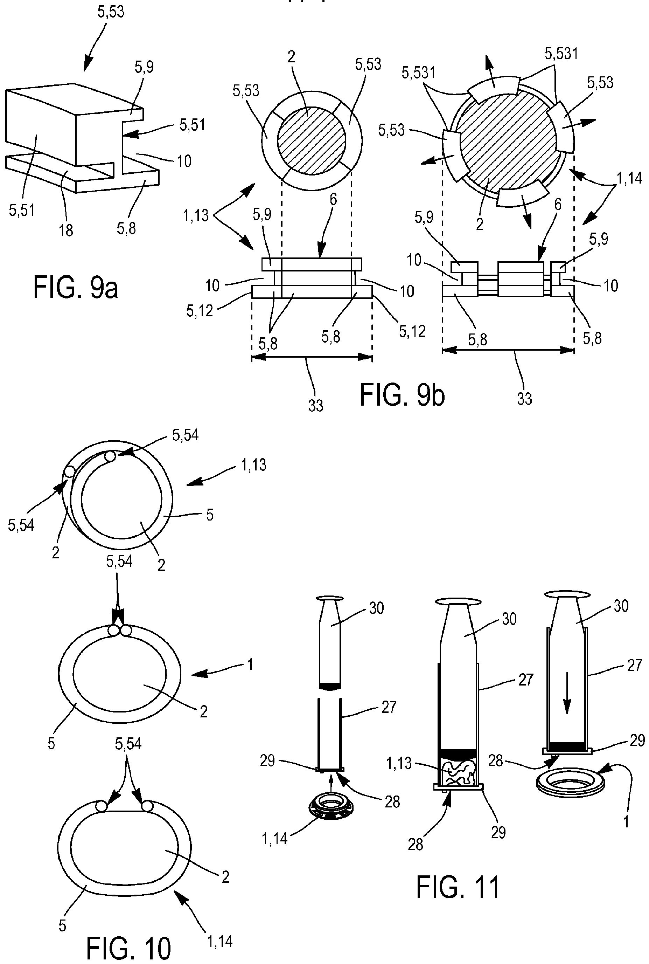

In a second embodiment depicted in FIGURES 9, the frame 5 comprises portions 53 separated from one another.

According to the second embodiment, in the retracted state 13, only a small part of the center of the window 2 is not covered by the portions 53. Each of two faces 531, so-called lateral faces 531, of a portion 53, so-called first portion, is in contact with one lateral face 531 of each of two other portions 53 adjacent to said first portion 53.

According to the second embodiment, in the extended state 14, a large part of the window 2 is not covered by the portions 53. Each portion 53 is separated from its two adjacent portions 53.

According to a first alternative of the second embodiment, the window 2 is flexible. The frame 5 may be rigid or flexible.

According to the first alternative, when no external force is applied to the IID 1, or to a part of the IID 1, the IID 1 tends to be in its retracted state 13. In the extended state of the IID 1, the apparent size of the window 2 is higher than the minimum apparent size of the window 2 for which the IID 1 is in its retracted state 13 and smaller than the maximum apparent size of the window 2. When the window 2 is flexible and the IID 1 is in its retracted state 13, the window 2 is folded.

According to the first alternative, the window 2 is rigid. The frame 5 may be rigid or flexible.

According to the second alternative, the IID 1 exhibits no idle state. When no external force is applied to the IID 1, or to a part of the IID 1, the IID 1 tends to stay in the state wherein it has been brought.

According to the second alternative, when the frame 5 is flexible, the apparent size of the window 2 is higher than the minimum apparent size of the window 2 for which the IID 1 is in its retracted state 13 and smaller than the maximum apparent size of the window 2 for which the IID 1 is in its extended state 14.

According to the second alternative, when the frame 5 is rigid, each portion 53 comprises a housing, or a slot, 18 in which the window 2, or a part of the window 2, is inserted.

According to the second alternative, when the frame 5 is rigid, the housings 18 may be arranged for the portions 53 to be moved relative to the window 2 through sliding of the window 2 inside the housings 18.

According to the second alternative, when the frame 5 is rigid:
- the window 2 is fully inserted in the housings 18 when the IID 1 is in its retracted state 13,
- the window 2 is partially inserted in the housings 18 when the IID 1 is in its extended state 14.

According to the second alternative, when the frame 5 is rigid; when no external force is applied to the IID 1, or to a part of the IID 1, the IID 1 tends to stay in the state wherein it has been brought.

In a third embodiment, depicted in FIGURES 10, the frame 5 comprises two end edges, or two end sides, 54 and the frame 5 is flexible so that the two end edges 54 are mobile relatively from one another.

The frame 5 is a leaf-spring that tends to bring the IID 1 in the extended state 14 when no external force is applied to the IID 1 or to a part of the IID 1.

According to a first alternative of the third embodiment, the window 2 is flexible. The window 2 is stuck or glued to, or molded or overmolded on, the frame 5. In the extended state 14 of the IID 1, the apparent size of the window 2 is higher than the minimum apparent size of the window 2 when the IID 1 is in its retracted state 13 and smaller than the maximum apparent size of the window 2.

According to a second alternative of the third embodiment, the window 2 is rigid and the frame 5 comprises a housing, or a slot, 18 in which the window 2, or a part of the window 2, is inserted. The housing 18 is arranged for the portions 53 to be moved relative to the window 2 through sliding of the window 2 inside the housing 18. When no external force is applied to the IID 1, or to a part of the IID 1, the IID 1 tends to be in its extended state 14 which is its idle state. The window 2 is then partially inserted in the housing 18 and the apparent size of the window 2 is maximum. When the IID 1 is in its retracted state 13, the window 2 is fully inserted in the housing 18 and the apparent size of the window 2 is minimum. In the retracted state 13 of the IID 1, an outer edge surface s1 of the window 2 is covered by the frame 5. In the extended state 14 of the IID 1, an outer edge surface s2 of the window 2, smaller than si, is covered by the frame 5.

In a fourth and preferred embodiment, illustrates in particular FIGURES 1a, 1b, 1c, 2a, 2b and 5, the frame 5 and the window 2 are flexible. The frame 5 and the window 2 are elastic and reversibly deformable in any directions.

The frame 5 and the window 2 are made of flexible materials. For instance, the frame 5 is made of butadiene-styrene copolymer and the window 2 is made of nylon.

Preferably, the frame 5 and the window 2 are made of PDMS.

More preferably, the frame 5 and the window 2 form a single piece of PDMS.

When no external force is applied to the IID 1, or to a part of the IID 1, the IID 1 tends to be in its extended state 14 which is its idle state. In the extended state of the IID 1, the apparent size of the window 2 is comprised between the minimum apparent size of the window 2 when the IID 1 is in its retracted state 13 and the maximum apparent size of the window 2.

The retracted state 13 of the IID 1 is depicted in FIGURE 1c. In its retracted state 13, the IID 1 is folded. The IID 1 can be folded in a lot of different other ways.

When the window 2 is made of PDMS, the window 2 is optically transparent and possesses refractive index at least similar than those of glasses that are usually used. However, such window 2 can exhibit thinner thickness and better transmittance than those of glasses. This improves the depth of imaging. The thickness of the window 2 is comprised between 5 and 500 µm.

The more parts of the IID 1 are made of polymer, in particular of elastomer, the lighter is the IID 1 compared to titanium and glass that are usually used. Thus, an IID 1 entirely made of polymer may does not exert a significant pulling force on the skin of the animal located above the inner bulge 8 and thus limit inflammatory reaction. This allows decreasing the skin degradation and prevents the IID 1 detachment.

The more parts of the IID 1 are made of polymer, in particular of elastomer, the more soft and flexible is the IID 1. Thus, a IID 1 entirely made of polymer is also much more soft and flexible compare to titanium and glass, thus allowing the IID 1 to fit the skin of the animal and to decrease pulling forces on the tissue during animal movement and growth. This also allows manual stretching of the window 2 and/or of any part of the IID 1 during imaging.

After the IID 1 has been implanted, the outer bulge 9 prevents the skin to overlay the window 2 when the animal is healing.

Moreover, elastomers can be biocompatible and, depending on their composition, can limit the immune response and fibrosis. When the window 2 is made of elastomer, it allows injecting substances in the organ or the part of the animal to be imaged.

All this features combined, avoid resorting to sutures. Use of sutures necessarily leads to drawbacks such as inflammation, fibrosis and infection. According to the invention, after the IID 1 has been implanted, the skin of the animal firmly encircles the groove 10 and forms a hermetic barrier with the exterior.

As illustrated in FIGURES 1 and 5, the inner bulge 8 comprises a coating 19 to promote the healing of the animal. The inner bulge 8 also comprises holes 20 to promote the healing of the host. The holes 20 enables embedding and fusion of the inner bulge 8 through healing of the skin of the animal.

As illustrated FIGURE 4, the outer bulge 9 comprises means for protecting the IID 1 against animal degradations. As illustrates FIGURE 4a, the means for protecting IID 1 against animal degradations comprises an abrasion resistant shield 21. As non-limiting example, this shield 21 can be made of polymer such as Polyamide or Polyethylene terephthalate or Polyoxymethylene or poly tetrafluoroethylene or filled nylon or Polyurethane or PDMS. Such shield 21 being more rigid, the shield 21 can be held on the outer bulge 9 through a holding device 22. As a non-limiting example, the holding device 22 can consist in holding pins 22 that are linked or part of the frame 5. Thus, in order to restore the flexibility of the IID 1 after few days of implantation, the shield 21 can be released through removing the holding pins 22 few days after implantation as soon as the skin has healed.

As illustrates FIGURE 4b, the means for protecting IID 1 against animal degradations can also comprise degradable distracting structure 23 to attract animal attention. This degradable distracting structure 23 may be combined with the shield 21. Degradable distracting structure 23 seeks to prevent the outer bulge 9 and the IID 1 from degradation. Degradable distracting structure 23 is deliberately visible and reachable by the animal. As a non-limiting example, this structure 23 can be wires 23 made of an elastomer material.

As depicted in FIGURE 6, the frame 5 comprises a protrusion 24 extending beyond the window 2 towards the area to be imaged. The protrusion 24 comprises a housing 25 arranged to immobilize and conserve an organ to be imaged. The housing 25 is adjacent to the window 2. The protrusion 24 also comprises an opening 26 extending from the housing 25 beyond an end of the protrusion 24 located on the side of the protrusion 24 opposite to the window 2. The opening 26 is a slot. The housing 25 is arranged for the organ to be imaged to be inserted in it. As illustrated in FIGURE 6b, the organ can be the small intestine 31 of the animal. The opening 26 is arranged to provide a way for other organs, such as blood vessels and nerves, to connect the small intestine to the animal.

The sizes and geometry of the parts 2, 5, 51, 52, 53, 54, 6, 8, 9, 10, 11, 12, 15, 16, 17, 18, 19, 20, 21, 22, 23, 24, 25, 26 of the IID 1 can be tailored. As illustrated in FIGURE 5, the shape of the outer bulge 9 can be flat or rounded depending on, among other shapes, the implantation site. The ratio between the size of the inner bulge 8 and the size of the outer bulge 9 according to a direction normal to the imaging direction 71 can vary, as appropriate.

In order to decrease the weight of the IID 1 and to limit damages caused by the animal attempts to remove the IID 1, the thickness of the bulge 9 is thin, for example twice the thickness of the skin, for instance between 500 µm and 3 mm. The thickness of the outer bulge 9, and so the total thickness of the IID 1, is as small as possible to limit grips for the animal to attempt removing the IID 1. The number of holes 20 in the inner bulge 8 is high and the size of the holes 20 is small.

As non-limiting example, the total thickness of the IID 1 is 3mm, the radius of the outer bulge 9 is 14 mm and the radius of the inner bulge 8 is 16 mm.

The IID according to the invention is used for preclinical testing or preclinical study or in vivo drug screening. As non-limiting example, the preclinical study may be dedicated to therapeutic efficacy or toxicity assay of a compound.

According to a fifth embodiment, there is also provided a method for implanting an IID 1 according to the invention. The IID 1 may comprise any combination of any feature, improvement, alternative or embodiment of the IID 1 according to the invention. For the sake of understanding and clarity, the IID 1 according to the fourth embodiment has been chosen to illustrate the method but any embodiment of the IID 1 according to the invention is suitable for the implementation of the method.

With reference to FIGURE 10, the method comprises the steps of retracting the IID 1 at least in part according to a direction normal to the imaging direction 71. This step consists in reversibly decreasing the surface 33 defined by a perimeter in which the IID 1 is inscribed. Then, the IID 1 in its retracted state 13 is loaded into a pipe 27 through an opening 28 of the pipe 27. After being loaded in its retracted state 13 into the pipe 27, the IID 1 closes the pipe 27. According to the embodiment, the pipe 27 is a syringe tube 27 having a diameter which is at least equal to, preferably lower than, the diameter of the IID 1.

After being loaded in its retracted state 13 into the pipe 27, the opening 28 is arranged into an incision site of the skin of the animal that faces the organ of the animal to be imaged. This step consists in inserting a rounded rim 29 of the pipe 27 into the incision site of the skin. The rounded rim 29 abuts on outer edges of the opening 28. The rounded rim 29 is arranged between the area to be imaged and the skin.

A pressure is then applied into the pipe 27. According to the embodiment, said pressure is applied through a syringe piston 30 inserted into the syringe tube 27. The pressure is directed towards the opening 28 so that the IID 1 is release from the pipe 27. The releasing of the IID 1 allows the skin of the animal to firmly encircle the groove 10 of the IID 1. The window 2 faces the area to be imaged.

In a preferred embodiment, the method further comprises the step of intravital imaging of the area to be imaged.

According to a sixth embodiment, there is also provided a method for preclinical testing or preclinical study or in vivo drug screening, said method comprising any combination of any feature, improvement, alternative or embodiment of the method according to the fifth embodiment of the invention. As non-limiting example, the method may be dedicated to the preclinical study of one organ or tissue of an animal or to the preclinical study of the therapeutic efficacy or toxicity assay of a compound.

In a preferred embodiment, there is provided a method for preclinical study of a candidate compound in at least one organ or tissue in an animal, said method comprising steps of implanting the IID according to the invention.

The IID 1 may comprise any combination of any feature, improvement, alternative or embodiment of the IID 1 according to the invention.

In a preferred embodiment, the method comprises the steps of:
- PDX genetic modification with specific fluoreporter, for instance pathways, apoptosis, proliferation, invasion, and/or
- intravital imaging of the organ and/or tissue and/or exogenous material, and/or
- therapeutic immune cell labeling, for instance immunotherapy assays, and/or
- xenograft of exogenous material, and/or
- administering the candidate compound to the animal.

The invention is not restricted to embodiments described above and numerous adjustments may be achieved within the scope of the invention.

Thus, in combinable alternatives of previous embodiments:
- according to the invention, the size of the opening 6, according a direction normal to the direction whereby the opening 6 is extending, the direction whereby the opening 6 is extending being called imaging direction 71, is larger than the size of the window 2 according to a direction normal to the imaging direction 71, and/or
- according to the invention, when no external force is applied to the IID 1, or to a part of the IID 1, the IID 1 exhibits an idle state that tends to bring the IID 1in its retracted state 14, and/or
- according to the invention, when the idle state of the IID 1 is its retracted state 14 and when an external force that brings the IID 1 in its extended state 13 is no longer applied, the IID 1 tend to return in its retracted state 14, and/or
- according to the invention, when the idle state of the IID 1 is its retracted state 14, when the IID 1 is implanted and when the tissue can cause the size of the window 2 to be different from the size of the window 2 when the IID 1 is in its retracted state 14, an external force may be applied during the imaging to:
   - increase the size of the IID 1, and/or
   - bring the IID 1 in its extended state 14, and/or
   - increase the size of the window 2 towards its apparent maximum size, and/or
- according to the invention, when no external force is applied to the IID 1, or to a part of the IID 1, the IID 1 exhibits no idle state, and/or
- according to the invention, after being implanted, a size of the window 2 and/or a size of the IID 1 can be reversely increase in order to increase the visible area of the organ to be imaged and/or to increase the accessible area of the organ, and/or
- according to the invention, the thickness of the window may be greater than 5 µm and/or may be lower than 500 µm, and/or
- according to the invention, the thickness of the window 2 may be greater than 10 or 15 or 20 or 25 µm and/or may be lower than 400 or 300 or 200 or 100 µm, and/or
- according to the improvement, the bulges 8,9 are extending outward on the side of the face 3 of the IID 1 that comprises the window 2, and/or
- according to the improvement, the angle β is different of the angle ϕ to enhance the clamping force, or the pinching force, applied by the bulges 8,9, on the tissue 34, and/or
- according to the improvement, before the IID 1 being implanted, when the angle β is different of the angle ϕ, a part of the faces of the bulges 8,9 facing each other are in contact so that the groove 10 forms a closed cavity, and/or
- according to the improvement, when the groove 10 forms a closed cavity, the bulges 8,9 are pulled away from one another during the implantation so that the tissue 34 could be inserted inside the groove 14, and/or
- according to the improvement, the angle β is lower than the angle ϕ to enhance:
   ∘ the overlaying, or the covering, of the bulges 8,9, and/or
   ∘ the clamping force, or the pinching force, applied by the bulges 8,9, on the tissue 34, and/or
- according to the improvement, the IID 1 comprises a component extending between the faces of the bulges 8,9 facing each other and exerting a spring force in order for the bulges 8,9 to act as a clamp, and/or
- according to a first alternative of the first embodiment, the portion 53 do not comprises a housing 17 in which the window 2, or a part of the window 2, is inserted, the window 2 is stuck or glued to, or molded or overmolded on, the portions 53, and/or
- according to the first alternative of the second embodiment, each portion 53 comprises a housing, or a slot, 18 in which the window 2, or a part of the window 2, is inserted, and/or
- according to the first alternative of the second embodiment, the housing 18 is the housing 17 of the first embodiment and the window 2 comprises the annular bead 16 held in the recess 172 arranged at the end of the slot 171, and/or
- according to the first alternative of the second embodiment, when the portions 53 comprise a housing, the window 2 is stuck or glued, or molded or overmolded, within the portions 53, and/or
- according to the first alternative of the second embodiment, when no external force is applied to the IID 1, or to a part of the IID 1, the IID 1 tends to be in its extended state 14, and/or
- according to the first alternative of the second embodiment, the housings 18 may be arranged for the portions 53 to be moved relative to the window 2 through sliding of the window 2 inside the housings 18, and/or
- according to the second alternative of the second embodiment, when the frame is flexible; the portions 5 comprise a housing 18 in which the window 2, or a part of the window 2, is inserted, and/or
- according to the second alternative of the second embodiment, the housing 18 is the housing 17 of the first embodiment and the window 2 comprises the annular bead 16 held in the recess 172 arranged at the end of the slot 171, and/or
- according to the second alternative of the second embodiment, the window 2 is stuck or glued, or molded or overmolded, within the housings 18 of the portions 53, and/or
- according to the second alternative of the second embodiment, the housings 18 may be arranged for the portions 53 to be moved relative to the window 2 through sliding of the window 2 inside the housings 18, and/or
- according to the first alternative of the third embodiment, the frame 5 comprises a housing, or a slot, 18 in which the window 2, or a part of the window 2, is inserted, and/or
- according to the third embodiment, the housing, or the slot, 18 is the housing 17 of the first embodiment or the housing 18 of the second embodiment and the window 2 comprises the annular bead 16 held in the recess 172 arranged at the end of the slot 171, and/or
- according to the first alternative of the third embodiment, the window 2 is stuck or glued, or molded or overmolded, within the housings 17, 18, and/or
- according to the first alternative of the third embodiment, the housings 17, 18 are arranged for the frame 5 to be moved relative to the window 2 through sliding of the window 2 inside the housings 17, 18, and/or
- according to a first improvement to the third embodiment:
   - each end edge 54 is elastically bound to the other end edge 54, through an elastic member, such as a spring or a rubber band, or
   - each end edge 54 is elastically bound to another part of the frame 5, through an elastic member, such as a spring or a rubber band, and/or
- according to the first improvement of the third embodiment, the elastic bound tends to bring the IID 1:
   - in the retracted state 13 wherein an apparent size of the window 2 is minimum, or
   - in the extended state 14 wherein an apparent size of the window 2 is maximum, and/or
- according to the third embodiment, in the retracted state 13, only a small part of the center of the window 2 is not covered by the frame 5, and/or
- according to the third embodiment, in the extended state 14, a large part of the window 2 is not covered by the frame 5, and/or
- according to the second alternative of the third embodiment, when the window 2 is rigid; when the IID 1 is in its retracted state 13, the window 2 exhibits its minimum apparent size, and/or
- according to the second alternative of the third embodiment, when the window 2 is rigid; when the IID 1 is in its extended state 14, the window 2 exhibits its maximum apparent size, and/or
- according to the invention, the inner bulge 8 comprises a functionalization and/or a surface treatment to promote the healing of the animal, and/or
- according to the invention, the inner bulge 8 comprises a functionalization and/or a surface treatment to promote the adhesion of the skin of the animal, and/or
- according to the invention, the groove 10 and/or the outer bulge 9 and/or the window 2 and/or the frame 5 or the entire IID 1 comprise:
   - a coating and/or a functionalization and/or a surface treatment and/or a filler material to promote the healing of the animal and/or to promote the adhesion of the skin of the animal, and/or
   - a surface pattern and/or an increase of surface roughness to promote the healing of the animal, and/or
- according to the invention, the inner bulge 8 and/or the groove 10 and/or the outer bulge 9 and/or the window 2 and/or the frame 5 or the entire IID 1 comprises surface pattern such as structures, such as micro and/or nanostructures, and/or
- according to the fifth embodiment, subsequently to the retracting step, the method comprises applying a clamping force on the skin 34 of the host located inside the groove 10 to prevent the skin 34 to be pulled out from the groove 10, and/or
- according to the fifth embodiment, subsequently to the retracting step, the method comprises applying a lifting force on the skin 34 of the host located in the vicinity of the outer periphery of the IID 1 so that a part of the skin 34 is positioned above and/or covers outer ends of the bulges 8,9, and so outer end of the IID 1.

Moreover, features, alternatives and embodiments of the invention may be associated if they are not mutually exclusive of each other.

## Claims

1. An Intravital Imaging Device (IID) comprising :
- a window forming at least a part of a face of the IID,
- a frame extending outward from a contour of the window, the frame comprising:
• an inner bulge located on a side of the IID comprising the window,
• an outer bulge located on a side of the IID opposite to the side of the IID comprising the window,
• a groove located between the outer and inner bulge,
the IID being **characterized in that**, it is arranged to reversibly decrease and/or increase a surface defined by a perimeter in which the IID is inscribed.

2. The IID according to claim 1, wherein:
- a non-zero angle is formed between a plane in which the contour of the window is inscribed and an axis along which the outer bulge is extending, and/or
- a non-zero angle is formed between the plane in which the contour of the window is inscribed and an axis along which the inner bulge is extending.

3. The IID according to claim 1 or 2, wherein a width of the groove decrease along a direction extending outward from a bottom of the groove.

4. The IID according to any of the preceding claims, wherein the inner and the outer bulge are arranged to act as a clamp.

5. The IID according to any of the preceding claims, wherein a surface defined by a perimeter in which the inner bulge is inscribed is larger than a surface defined by a perimeter in which the outer bulge is inscribed or vice versa.

6. The IID according to any of the preceding claims, exhibits at least two states:
- a retracted state, and
- an extended state wherein an apparent size of the window is higher than an apparent size of the window when the IID is in its retracted state.

7. The IID according to any of the preceding claims, wherein the frame and the window are flexible.

8. The IID according to any of the preceding claims, wherein the frame and the window form a single piece.

9. The IID according to any of the preceding claims, wherein the inner bulge comprises:
- a coating and/or a functionalization and/or a surface treatment and/or a filler material to promote the healing of an host and/or to promote the adhesion of a tissue of the host, and/or
- a surface pattern and/or an increase of surface roughness to promote the healing of the host and/or to promote the adhesion of the tissue of the host.

10. The IID according to any of the preceding claims, wherein the outer bulge comprises means for protecting the IID against animal degradation.

11. The IID according to claim 10, wherein the means for protecting the IID against animal degradation comprises:
- an abrasion resistant shield, and/or
- a degradable distracting structure to attract animal attention.

12. The IID according to any of the preceding claims, comprises a housing located on the face of the IID comprising the window, the housing being arranged for:
- receiving an object of the host to be imaged, and/or
- immobilizing the object of the host to be imaged, and/or
- conserving the object of the host to be imaged.

13. Method for implanting an Intravital Imaging Device (IID) according to any of claims 1 to 12 or for preclinical testing or for preclinical study or for in vivo drug screening, the method comprises the steps consisting in:
- retracting the IID by reversibly decreasing a surface defined by a perimeter in which the IID is inscribed,
- loading the retracted IID into a pipe through an opening of the pipe,
- arranging the opening so that it faces an area of an host to be imaged,
- applying a pressure in the pipe, said pressure being directed towards the opening for releasing the IID from the pipe so that:
• a groove of the IID receives a tissue of the host, and
• a window of the IID faces the area to be imaged.

14. Method according to claim 13, comprising the step consisting in inserting a rounded rim of the pipe into the incision site of the tissue and arranging the rounded rim between the area to be imaged and the tissue; the rounded rim being abutted on outer edges of said opening.

15. Method according to claim any of claims 13 or 14, wherein subsequently to the retracting step:
- a clamping force is applied on the tissue of the host located inside the groove, and/or
- a lifting force is applied by the IID on the tissue of the host located outside the groove so that a part of the tissue of the host is positioned above and/or covers an outer peripheral portion of the IID.
